(19) **Europäisches Patentamt**

**European Patent Office**

**Office européen des brevets**

(11) **EP 0 728 730 B1**

(12) **EUROPÄISCHE PATENTSCHRIFT**

(45) Veröffentlichungstag und Bekanntmachung des Hinweises auf die Patenterteilung:
**07.03.2001 Patentblatt 2001/10**

(51) Int Cl.$^7$: **C07C 53/16**, C07C 51/377, C07C 51/487

(21) Anmeldenummer: **94119029.0**

(22) Anmeldetag: **02.12.1994**

(54) **Verfahren zur Herstellung von Monochloressigsäure**

Process for preparing monochloroacetic acid

Procédé de préparation d'acide monochloroacétique

(84) Benannte Vertragsstaaten:
**AT BE CH DE DK ES FR GB GR IE IT LI LU MC NL PT SE**
Benannte Erstreckungsstaaten:
**SI**

(43) Veröffentlichungstag der Anmeldung:
**28.08.1996 Patentblatt 1996/35**

(73) Patentinhaber:
• **Salzgitter Anlagenbau GmbH**
  **38239 Salzgitter (DE)**
• **Scientific Research Institute " Syntez"**
  **109432 Moskau (RU)**

(72) Erfinder:
• **Nikolaevich, Zanaveskin Leonid, Wissenschaftliches**
  **109432 Moskau (RU)**
• **Nikolaevich, Bulanov Viacheslav, Wissenschaftlich.**
  **109432 Moskau (RU)**
• **Georgievna, Rumianceva Nina, Wissenschaftliches**
  **109432 Moskau (RU)**
• **Olegovich, Bobkov Alexej, Wissenschaftliches**
  **109432 Moskau (RU)**
• **Alexejevich, Grischin Viacheslav, "Mittelvolzhsker**
  **446100 Tschapajevsk (RU)**
• **Alexejevich, Fomin Vladimir, "Mittelvolzhsker**
  **446100 Tschapajevsk (RU)**
• **Träger, J. A.**
  **111531 Moskau (RU)**
• **Sobotta, Georg, Dr.**
  **D-38300 Wolfenbüttel (DE)**
• **Entner, Roland, Dipl.-Chem.**
  **D-38274 Elbe (DE)**

(74) Vertreter: **Köckeritz, Günter et al**
**Preussag AG**
**Patente & Lizenzen**
**Postfach 61 02 09**
**30602 Hannover (DE)**

(56) Entgegenhaltungen:
EP-A- 0 537 838     DE-A- 1 816 931
DE-B- 1 201 326     FR-A- 2 645 531

• DATABASE WPI Section Ch, Week 8403, 29. Februar 1984 Derwent Publications Ltd., London, GB; Class E, AN 84-016387 'PURIFICN.OF MONOCHLOROACETIC ACID-BY CATALYTIC HYDROGENATION ON PALLADIUM CHARCOAL IN PRESENCE OF SURFACE ACTIVE SODIUM MONOCHLOROACETATE.' & SU-A-104 345 (KUIBYSHEV POLY) 17. April 1981

**Beschreibung**

Die Erfindung betrifft die Verbesserung des (MCHES) MonochloressigsäureVerfahrens und kann in der chemischen Industrie verwendet werden.

MCHES ist ein wertvolles Produkt für die Herstellung von Karboxymethylzellulose, diversen Pestiziden und Arzneimitteln.

Gewonnen wird die Monochloressigsäure mittels indirekter Hydratisierung von Trichloräthylen

$$CHCl{\equiv}{\equiv}{\equiv}CCl_2 + 2H_2O \text{ -----> } CH_2ClCOOH + 2\,HCl$$

oder durch Oxidation von Monochlorazetaldehyd oder Äthylenchlorhydrin, zum Beispiel

$$CH_2ClCHO + 0,5\,0_2 \text{ -----> } CH_2ClCOOH$$

Aus der US-A-2 863 917 ist ein Verfahren zur Reinigung technischer MCHES von der darin als Nebenprodukt enthaltenen DCHES mittels einer Selektiv-Chlorierung (Entchlorung) bekannt. Die Durchführung des Hydrierprozesses der MCHES unter Bedingungen, wo der Katalysator im Reaktionsvolumen frei verteilt ist, führt zu einem wesentlichen Abrieb der Katalysatorpartikel wegen deren intensiver Bewegung bei der Zuführung von gasförmigem Wasserstoff.

Die DE-A-18 16 931 beinhaltet ein Verfahren zur Reinigung von MCHES mittels Hydrierung in Gegenwart eines suspendierten, feinkörnigen Katalysators auf Basis Siliziumoxid oder Aktivkohle. Der Katalysator soll dabei in ständiger Bewegung gehalten werden, wobei ein Katalysatorabrieb erfolgt, was zu dessen Verlust und zur Verschmutzung der Reaktionsprodukte führt.

Das dem anzumeldenden Verfahren am meisten entsprechende Verfahren zur Herstellung von Monochloressigsäure beinhaltet eine kontinuierliche, katalytische Flüssigphasenchlorierung der Essigsäure (ES) und die Abscheidung der Monochloressigsäure mittels Kristallisation und anschließender Zentrifugierung. Die von den Kristallen getrennte Flüssigphase (Mutterlauge) wird zum Chlorierungsstadium zurückgeführt.

Bei der bekannten Methode wird bei der Chlorierung von Essigsäure Essigsäureanhydrid als Katalysator eingesetzt. Die im Prozeß verlaufenden Reaktionen können wie folgt dargestellt werden:

$$(CH_3CO)_2O + HCl \text{ -----> } CH_3COCl + CH_3COOH$$

$$CH_3COCl + Cl_2 \text{ -----> } CH_2ClCOCl + HCl$$

$$CH_2ClCOCl + CH_3COOH \text{ -----> } CH_2ClCOOH + CH_3COCl$$

usw.

Als das wichtigste Nebenprodukt gilt die Dichloressigsäure (DCHES), welche bei der Chlorierung von Monochlorazetylchlorid entsteht:

$$CH_2ClCOCl + Cl_2 \text{ -----> } CHCl_2COCl + HCl$$

$$CHCl_2COCl + CH_3COOH \text{ -----> } CHCl_2COOH + CH_3COCl$$

Die kontinuierliche Chlorierung der Essigsäure wird durchgeführt bei 100 bis 120 Grad Celsius im Endproduktmedium, welches 75 gew. % MCHES (Monochloressigsäure), 18 gew. % Essigsäure und 7 gew. % Dichloressigsäure enthält.

Das Chlorierungsprodukt wird bei 50 Grad Celsius dem Kristallisationsstadium zugeführt. Die Kristallisation wird durchgeführt bei Abkühlung des Produktes bei 20 bis 25 Grad Celsius. Im Ergebnis bildet sich eine Suspension von MCHES-Kristallen im Gemisch mit Essigsäure und Dichloressigsäure.

Danach wird die Suspension einer Trennung unterzogen, zum Beispiel mittels Zentrifugierung. Die feste Phase ist das handelsübliche Produkt, die Flüssigphase wird zur Chlorierung geleitet.

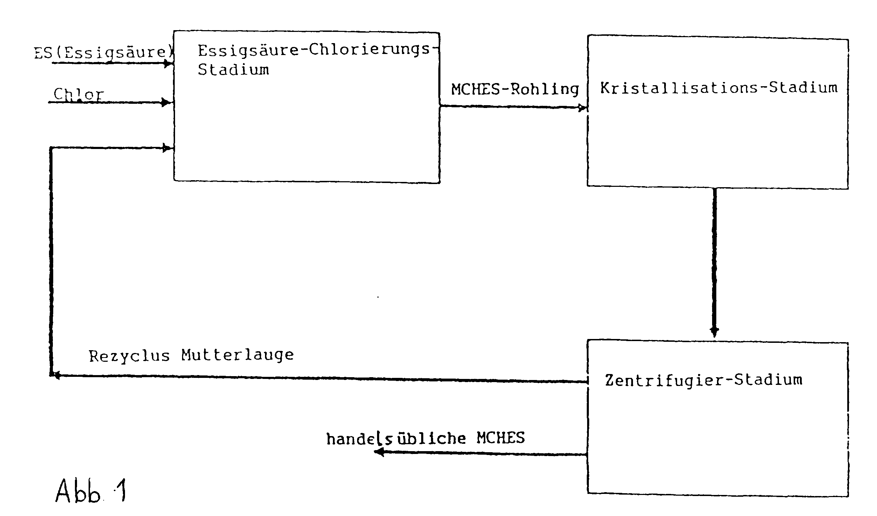

Auf der Abbildung 1 ist der Ablaufplan des bekannten MonochloressigsäureVerfahrens dargestellt.

Aus der Beschreibung und dem Ablaufplan des bekannten MCHES-Verfahrens ist ersichtlich, daß die Zuführung von Mutterlauge im Chlorierungsstadium zu einer Ansammlung von DCHES (Dichloressigsäure) im Schema führt, was die Ableitung eines Teiles der Mutterlauge zur Vernichtung erforderlich macht. Die hohe Konzentration der DCHES setzt außerdem die Leistung des Kristallisationsstadiums herab.

Als eine Unzulänglichkeit beim bekannten MCHES-Verfahren gilt daher die Notwendigkeit der Vernichtung eines Teiles der Mutterlauge, was zu einer hohen Verbrauchskennziffer beim Rohstoff führt sowie eine niedrige Leistung beim Kristallisationsstadium wegen des hohen DCHES-Gehaltes im Rohling der Monochloressigsäure.

Das Ziel dieser Erfindung ist die Herabsetzung der Rohstoffverbrauchskennziffer und Erhöhung der Leistung bei der MCHES-Kristallisation.

Erfindungsgemäß wird dieses Ziel durch die Merkmale des Anspruchs 1 gelöst. Vorteilhafte Ausführungen der Erfindung sind in den Ansprüchen 2 bis 5 enthalten. Demnach beinhaltet die Erfindung ein Verfahren zur Herstellung von Monochloressigsäure (MCHES) durch Chlorierung von Essigsäure, wobei ein MCHES-Rohling erzeugt wird und aus dem Rohling durch Kristallisation und Zentrifugierung MCHES abgetrennt wird, dadurch gekennzeichnet, dass vor der Kristallisation eine katalytische Hydrogenolyse durchgeführt, wobei diese in einem mit Gasaufzug (Airlift) ausgestatteten Apparat oder an einem Festbettkatalysator im Dünnschichtbetrieb vorgenommen wird.

Nach weiteren bevorzugten Merkmalen der Erfindung wird in der Hydrogenolyse ein Molverhältnis DCHES : $H_2$ = 1 : 7 eingestellt, wobei die Hydrogenolyse bei Temperaturen zwischen 125 bis 140 °C durchgeführt wird.

Nach einem anderen bevorzugten Merkmal der Erfindung erfolgt der Hydrogenolyse-Prozeß in einem Festbett-Katalysator im Dünnschichtbetrieb bei einer Zufuhrgeschwindigkeit des MCHES-Rohlings zwischen 0,049 und 1,24 m/s, insbesondere zwischen 0,1 und 0,5 m/s.

Das gesetzte Ziel wird erreicht durch Einführung eines Hydrogenolysestadiums des MCHES-Rohlings nach dem Chlorierungsstadium und durch Weiterleitung des Produktes nach dem Hydrierungsstadium zum Kristallisationsstadium.

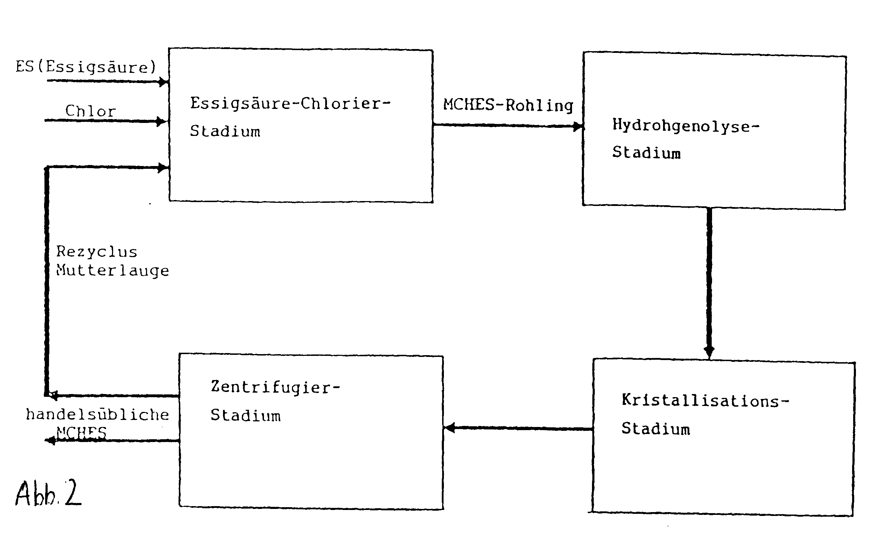

Auf Abbildung 2 ist der Ablaufplan des vorgeschlagenen Monochloressigsäure-Verfahrens dargestellt.

Das erzielte Ergebnis - bestehend aus Herabsetzung der Verbrauchskennziffer nach dem Rohstoff und Erhöhung der Leistung des Kristallisationsstadiums - wird durch Folgendes gewährleistet: die Einführung des Stadiums der katalytischen Hydrogenolyse des MCHES-Rohlings nach dem Chlorierungsstadium ermöglicht die Herabsetzung der DCHES-Menge im Rohling von 7 gew. % auf 1 bis 1,5 gew. % und schließt somit die Notwendigkeit der Ableitung der Mutterlauge zur Vernichtung aus, das heißt, mit dem Verfahren wird die Ansammlung von DCHES ausgeschlossen.

Die Herabsetzung des DCHES-Gehalts im MCHES-Rohling wird außerdem bei sonstigen gleichen Bedingungen zur Erhöhung der Leistung im Kristallisationsstadium führen.

In Abbildung 2 ist das Blockschema des vorgeschlagenen Verfahrens zur Herstellung von Monochloressigsäure dargestellt. Das erzielbare Ergebnis, welches in der Senkung der Verbrauchswerte bezogen auf den Rohstoff und in der Erhöhung der Leistung im Kristallisationsstadium besteht, wird sichergestellt durch Einführung der nachfolgenden Stufe einer katalytischen Hydrogenolyse (substitutive Hydrierung) des MCHES-Zwischenproduktes nach der Chlorierungsstufe und ermöglicht die Herabsetzung der Menge des DCHES für die Zwischenstufe von 7 gew. % auf 1 bis 2,5 gew. %. Somit wird die Notwendigkeit der Ableitung der Mutterlauge zur Vernichtung ausgeschlossen. (Im Blockschema ist die Ansammlung von DCHES ausgeschlossen.) Die Herabsetzung des DCHES-Gehaltes im MCHES-Zwischenprodukt wird außerdem bei sonst gleichen Bedingungen zu einer Steigerung der Leistung im Kristallisationsstadium führen. Als Katalysator wird 0,5 bis 2 gew. % Palladium auf Aktivkohle verwendet.

Die vorgeschlagene technische Lösung wird durch folgende Beispiele illustriert:

Beispiel 1 (bekanntes Verfahren)

In einem Reaktor (Kolonnentyp) mit Mantel wird bei einer Temperatur von 110 Grad Celsius in der Flüssigphase ein katalytischer (bei Zugabe von Essigsäureanhydrid 33,6 g/h) Chlorierungsprozeß der Essigsäure mittels gasförmigem Chlor durchgeführt. Der Chlorierungsprozeß wird bei einem 10 %igen Mol-Überschuß an Chlor durchgeführt.

Das Kristallisationsstadium ist ein volumetrischer Apparat, in dessen Mantel Kältemittel zugeführt wird. In den Kristallisatoren erfolgt in 12 Stunden die Abkühlung des MCHES-Rohlings von 50 auf 20 Grad Celsius.

Nach Abschlulß des Kristallisationsprozesses leitet man die gewonnene Suspension zum Zentrifugieren. Danach wird die Qualtität und Zusammensetzung der Mutterlauge ermittelt sowie die Menge und Zusammensetzung der handelsüblichen MCHES.

Nach den Versuchsergebnissen ermittelt man die Verbrauchskennziffer des Rohstoffes und die Ausbeute an Zielprodukten im Kristallisationsstadium.

Die Bedingungen für die Durchführung des Versuches und die erzielten Ergebnisse sind in der Tabelle 1 aufgeführt.

Beispiel 2

Die Chlorierung der Essigsäure wird analog der im Beispiel 1 beschriebenen Methode durchgeführt.

Den bei der Chlorierung gewonnenen MCHES-Rohling leitet man zum Stadium der katalytischen Hydrogenolyse. Der Prozeß wird durchgeführt in einem volumetrischen Apparat, in welchen 40 g Katalysator mit 1 gew. % Palladium auf Aktivkohle eingefüllt wird, bei einer Temperatur von 140 Grad Celsius und einem Molverhältnis DCHES : $H_2$ = 1:7.

Nach dem Hydrogenolysestadium wird das Produkt zum Kristallisationsstadium geleitet. Der Kristallisationsprozeß wird analog dem im Beispiel 1 beschriebenen durchgeführt.

Nach den Versuchsergebnissen werden die Verbrauchskennziffer für Rohstoff und die Ausbeute des Zielproduktes im Kristallisationsstadium ermittelt.

Die Bedingungen für die Durchführung des Versuches und die erzielten Ergebnisse sind in der Tabelle 1 aufgeführt.

Die Analyse der in der Tabelle 1 aufgeführten Daten zeigt, daß die Verwendung der Hydrogenolysemethode beim MCHES-Rohling nach dem Chlorierungsstadium es ermöglicht, die Verbrauchskennziffer bei Rohstoff, Essigsäure und Chlor wesentlich herabzusetzen sowie die Leistung des Kristallisationsstadiums zu erhöhen.

Als ein wesentliches Unterscheidungsmerkmal des vorgeschlagenen Monochloressigsäureverfahrens ist die Verwendung des Stadiums der katalytischen Hydrogenolyse beim MCHES-Rohling zwischen den Stadien der Chlorierung und der Kristallisation zu nennen.

Als Mangel des Verfahrens kann der hohe Verbrauch an Palladium-Katalysator (Katalysatorverbrauch 1,5 kg je 1 Tonne Produkt) und die Verunreinigung der gereinigten MCHES durch diesen, sowie eine niedrige Selektivität des Prozesses angesehen werden. Ein dermaßen hoher Verbrauch an Katalysator, welcher z. B. 2 gew. % Palladium auf Aktivkohle enthält, wird durch deren Abnutzung während der Durchsprudelung des Reaktors mit Wasserstoff erklärt: der Katalysator wird vom Gasstrom ergriffen, es kommt zu einer Abnutzung durch gegenseitige Reibung der Katalysatorpartikel und ein nachfolgendes Mitreißen mit dem Reaktionsgemisch bei dessen Ablauf. Die niedrige Prozeßselektivität wird damit erklärt, daß während des Hydrogenolyseprozesses nach dem bekannten Verfahren einer Wiederherstellung nicht nur die DCHES sondern auch die MCHES unterzogen wird:

$$CH_2ClCOOH + H_2 \text{ -----> } CH_3COOH + HCl$$

Das weitere Ziel dieser Erfindung ist die Herabsetzung des Katalystorverbrauches im Prozeß der MCHES-Reinigung mittels Hydrogenolyse und die Erhöhung der Prozeßselektivität.

Das gesetzte Ziel wird erreicht mittels Durchführung des Hydrogenolyseprozesse in einem mit Gasaufzug (Airlift) ausgestatteten Apparat. Der Gasaufzug stellt ein Rohr dar mit einer Einführung für Wasserstoff im unteren Teil und ein Mischer, in welchem sich ein Gas-Flüssigkeits-Gemisch aus Wasserstoff und MCHES bildet. Durch die Verringerung des spezifischen Gewichtes des Gas-Flüssigkeits-Gemisches steigt dieses im Rohr nach oben und reißt die MCHES aus dem Unterteil des Reaktors mit. Am Ausgang aus dem Rohr erfolgt die Trennung des Gas-Flüssigkeit-Gemisches. Hierbei wird der nicht in Reaktion getretene Wasserstoff mit den Abgasen aus dem Reaktor entfernt, die MCHES wird in den oberen Reaktorteil zurückgeführt.

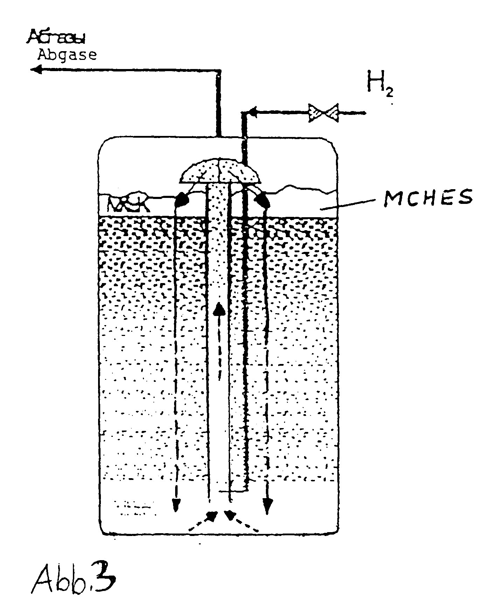

Zwischen dem oberen und unteren Teil des Rohres (Airliftes), in welches der Wasserstoff gelangt, ist der Reaktor mit dem Hydrogenolysekatalysator gefüllt. Durch den vom Airlift entstehenden Effekt gewährleistet man die Zirkulation der Reaktionsmasse im Reaktorvolumen von oben nach unten; im Ergebnis wird die Katalysatorschicht vom Strom dieser Reaktionsmasse zum Tragrost gepreßt. Der Hydrogenolyseprozeß verläuft auf Kosten des im Reaktionsgemisch beim Durchlaufen des Airliftes aufgelösten Wasserstoffes. Zum besseren Verständnis der vorgeschlagenen (angebotenen) technischen Lösung wird in Abbildung 3 ein Schema des bevorzugten Hydrogenolysereaktors für MCHES gezeigt.

Der positive Effekt, bestehend in der Herabsetzung des Katalysatorverbrauches und der Erhöhung der Selektivität, wird durch den Ausschluß der Wasserstoffzufuhr in die Katalysatorschicht erreicht.

Die vorgeschlagene technische Lösung wird durch folgende Beispiele illustriert:

Beispiel 3

In den Reaktor mit einem Volumen von 150 ml gibt man 30 g Katalysator mit 1 gew. % Palladium auf Aktivkohle und 120 g MCHES-Rohling mit 8,6 gew. % DCHES. Nach dem Erwärmen der Reaktionsmasse bis 140 Grad Celsius wird Wasserstoff mit einer Geschwindigkeit von 6 l/h zugeführt. Nach 6 Stunden unterbricht man die Wasserstoffzufuhr. In der Reaktionsmasse geht der Gehalt an DCHES runter auf 1,18 gew. %, der Gehalt an Essigsäure beträgt 2,8 gew. % und der Katalysatorverbrauch betrug 1,5 g je 1 kg Reaktionsgemisch.

Beispiel 4

In den Reaktor mit einem Volumen von 150 ml gibt man 30 g Katalysator mit 1 gew. % Palladium auf Aktivkohle und 120 g MCHES-Rohling mit 8,6 gew. % DCHES. Nach dem Erwärmen der Reaktionsmasse bis 140 Grad Celsius wird Wasserstoff mit einer Geschwindigkeit von 9 l/h zugeführt. Nach 6 Stunden unterbricht man die Wasserstoffzufuhr. In der Reaktionsmasse geht der Gehalt an DCHES runter auf 1,08 gew. %, der Gehalt an Essigsäure beträgt 3,7 gew. % und der Katalysatorverbrauch betrug 1,65 g je 1 kg Reaktionsgemisch.

Beispiel 5

In den Reaktor mit einem Volumen von 150 ml gibt man 30 g Katalysator mit 1 gew. % Palladium auf Aktivkohle und 120 g MCHES-Rohling mit 8,6 gew. % DCHES. Nach dem Erwärmen der Reaktionsmasse bis 140 Grad Celsius wird Wasserstoff mit einer Geschwindigkeit von 6 l/h dem Airlift-System zugeführt, welches ein durch die Katalysatorschicht führendes Rohr darstellt (analog dem oben Beschriebenen). Nach 6 Stunden unterbricht man die Wasserstoffzufuhr. In der Reaktionsmasse geht der Gehalt an DCHES runter auf 1,02 gew. %, der Gehalt an Essigsäure beträgt 1,56 gew. % und der Katalysatorverbrauch betrug 0,05 g je 1 kg Reaktionsgemisch.

Beispiel 6 (Airlift)

In den Reaktor mit einem Volumen von 150 ml gibt man 45 g Katalysator mit 1 gew. % Palladium auf Aktivkohle und 120 g MCHES-Rohling mit 8,6 gew. % DCHES. Nach dem Erwärmen der Reaktionsmasse bis 140 Grad Celsius wird Wasserstoff mit einer Geschwindigkeit von 6 l/h dem Airlift-System zugeführt. Nach 6 Stunden unterbricht man die Wasserstoffzufuhr. In der Reaktionsmasse geht der Gehalt an DCHES runter auf 0,95 gew. %, der Gehalt an Essigsäure beträgt 1,64 gew. % und der Katalysatorverbrauch betrug 0,04 g je 1 kg Reaktionsgemisch.

Beispiel 7 (Airlift)

In den Reaktor mit einem Volumen von 150 ml gibt man 45 g Katalysator mit 1 gew. % Palladium auf Aktivkohle und 120 g MCHES-Rohling mit 8,6 gew. % DCHES. Nach dem Erwärmen der Reaktionsmasse bis 140 Grad Celsius wird Wasserstoff mit einer Geschwindigkeit von 9 l/h dem Airlift-System zugeführt. Nach 6 Stunden unterbricht man die Wasserstoffzufuhr. In der Reaktionsmasse geht der Gehalt an DCHES runter auf 0,92 gew. %, der Gehalt an Essigsäure beträgt 1,62 gew. % und der Katalysatorverbrauch betrug 0,05 g je 1 kg Reaktionsgemisch.

Als ein wesentliches spezifisches Merkmal des vorgeschlagenen Verfahrens gilt - Durchführung des Prozesses mit Verwendung des Airliftes und Zufuhr des gesamterforderlichen Wasserstoffes in dieses Airlift. Im Endergebnis der Airliftverwendung wird die Wasserstoffzufuhr in die Katalysatorzone ausgeschlossen, was den Katalysatorverschleiß auschließt und - folglich - zu dessen Verbrauchssenkung und Verbesserung der Fertigproduktqualität führt. Außerdem wird die Prozeßselektivität erhöht: eine wesentlich kleinere Menge MCHES wird zu Essigsäure.

Die aufgeführten Beispiel zeigen, daß die vorgeschlagene Methode zur Reinigung der MCHES es ermöglicht - bei der Aufrechterhaltung eines hohen Grades der Reinigung des Produktes von DCHES - den Verbrauch des Katalysators, welcher das teuere Metall Palladium enthält, wesentlich herabzusetzen, die Produktqualität duch Herabsetzung der Katalysatorpartikel im letzten sowie die Produktselektivität zu erhöhen.

Alternativ zur Verwendung eines Airliftes kann die Druchführung des Hydrogenolyseprozesses in einer Dünnschichtbetriebsweise im Apparat mit Festbettkatalysator, bei Berieselung der Katalysatorschicht mit MCHES-Rohling und Wasserstoffzufuhr in den Oberteil des Apparates erfolgen. Die fiktive (rechnerische) Zuführungsgeschwindigkeit für den MCHES-Rohling muß hierbei $4,9 * 10^{-2}$-1,24 m/s sein.

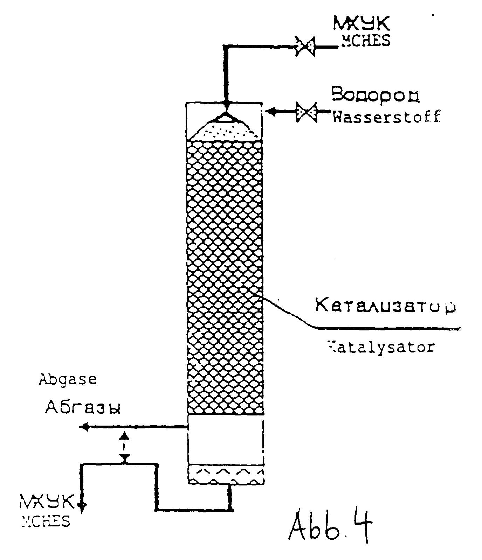

In Abbildung 4 ist das verfahrenstechnische Schema des Prozesses dargestellt.

Ein positiver Effekt beim vorgeschlagenen Verfahren, bestehend in der Herabsetzung des Katalysatorverbrauches, wird erreicht wegen der Durchführung eines Prozesses der Reinigung der MCHES von Beimischungen der DCHES (Dichloressigsäure) bei einer Dünnschichtbetriebsweise im Apparat, der analog einem Aufsatzapparat mit Füllmasse aufgebaut ist; als Füllmasse wird Palladiumkatalysator, zum Beispiel 1 % Palladium auf Aktivkohle verwendet. Dies begünstigt außerdem die Wasserstoffzuführung in das Oberteil des Apparates. Eine solche Zuführung von Reagenzien schließt die Möglichkeit einer Katalysatorpartikelbewegung aus und somit deren Abreibung und Mitreißen.

Die Notwendigkeit der Durchführung des MCHES-Reinigungsverfahrens bei einer fiktiven Zufuhrgeschwindigkeit von $4,9*10^{-2}$-1,24 m/s besteht darin, daß bei einer fiktiven Geschwindigkeit unter $4,9*-10^{-2}$ m/s einige Kataly-satorteile von der abfließenden MCHES-Dünnschicht frei werden, wodurch an diesem Katalysatorteil ein intensiver Dampfphasen-Hydrogenolyse-Prozeß der MCHES zu Essigsäure verläuft, das heißt, die Prozeßselektivität wird herabgesetzt.

Die Durchführung des Prozesses bei einer fiktiven Zufuhrgeschwindigkeit der MCHES von über 1,24 m/s führt zu einem Strahlabfließen auf der Katalysatoroberfläche und folglich zur Herabsetzung des Rohlingreinigungsgrades.

Die vorgeschlagene Erfindung wird durch folgende Beispiele illustriert:

Beispiel 8 (bekanntes Verfahren)

Einen vertikalen, zylindrischen Apparat beschickt man mit 80 g Katalystor, welcher 1 gew. % Palladium auf Aktivkohle enthält und 332 g (210 ml) MCHES-Rohling mit 8,6 gew. % DCHES. Nach dem Erwärmen der Reaktionsmasse bis 140 Grad Celsius wird Wasserstoff mit einer Geschwindikgeit von 6 l/h zugeführt. Nach 6 Stunden unterbricht man die Wasserstoffzufuhr. In der Reaktionsmasse geht der Gehalt an DCHES runter auf 1,18 gew. %, der Gehalt an Essigsäure (ES) beträgt 2,8 gew. %, und der Katalystorverbrauch betrug 1,5 g je 1 kg Reaktionsgemisch.

Beispiel 9 (Dünnschicht Betrieb)

In einen Reaktor, der einen vertikalen, zylindrischen Apparat mit variablem Durchmesser darstellt, gibt man 80 g Katalysator mit 1 gew. % Palladium auf Aktivkohle, und im oberen Reaktorteil verteilt man - mit einer Geschwindigkeit von 35 ml/h - gleichmäßig MCHES-Rohling, welcher 8,6 gew. % DCHES enthält, und es wird Wasserstoff mit einer Geschwindigkeit von 6,6 l/h zugeführt. Der Prozeß wird bei 140 Grad Celsius durchgeführt.

Die Bedingungen der Durchführung des Experimentes und die erzielten Ergebnisse sind in Tabelle 2 dargestellt.

Aus den in der Tabelle 2 aufgeführen Daten folgt, daß im gesamten dargestellten Intervall der fiktiven MCHES-Zufuhrgeschwindigkeiten man keinen Verbrauch an Palladiumkatalysator beobachten konnte. Somit ist das gesetzte Ziel der Erfindung im Teil der Minderung des Katalysatorverbrauches erreicht.

Bei einer fiktiven Geschwindigkeit für die Zufuhr des MCHES-Rohlings von $4,9*10^{-2}$ bis 1,24 m/s sind die Konzentrationen der DCHES und der Essigsäure (ES) niedriger als beim bekannten Verfahren.

Bei einer fiktiven Geschwindigkeit von über 1,24 m/s ist die Konzentration der DCHES höher, als beim bekannten Verfahren; bei einer fiktiven Geschwindigkeit von unter 0,049 m/s ist die Konzentration der Essigsäure höher als beim bekannten Verfahren. Folglich wird das gesetzte Ziel der Erfindung im Teil der Erhöhung des Prozeßselektivität erreicht im Intervall der Zufuhr des MCHES-Rohlings von $4,9*10^{-2}$-1,24 m/s.

Als ein wesentliches spezifisches Merkmal des vorgeschlagenen (angebotenen) MCHES-Reinigungsverfahrens gilt - Durchführung des Hydrogenolyseprozesses bei einer Folienbetriebsweise im Apparat mit Festbettkatalysator, unter Bedingungen der Berieselung der Katalysatorschicht mit MCHES-Rohling und Wasserstoffzufuhr in das Oberteil des Apparates. Hierbei muß die fiktive (aufgeführte) Zufuhrgeschwindigkeit für den MCHES-Rohling $4,9*10^{-2}$-1,24 m/s betragen.

Als besonders bevorzugt können Zufuhrgeschwindigkeiten von 0,5 bis 0,1 m/s angesehen werden, da bei diesen sowohl der DCHES-Gehalt als auch der ES-Gehalt günstige Werte annehmen.

Tabelle 1

| Chlorierungsstadium | Beispiel 1 | Beispiel 2 |
|---|---|---|
| Zuführung von $Cl_2$, g/h | 331.76 | 331.76 |
| Zuführung von ES (Essigsäure) g/h | 235.85 | 235.85 |
| Gewonnen an MCHES-Rohling, g/h | 396.00 | 396.00 |
| davon, g/h | | |
| MCHES | 297.00 | 297.00 |
| DCHES | 27.72 | 27.72 |
| ES | 71.28 | 71.28 |
| **Hydrogenolysestadium** | | |
| Hydrogenolyseprodukt, g/h | | 390.03 |
| davon, g/h | | |
| MCHES | | 313.35 |
| DCHES | | 5.40 |
| ES | | 71.28 |

Tabelle 1   (fortgesetzt)

| Chlorierungsstadium | Beispiel 1 | Beispiel 2 |
|---|---|---|
| **Kristallisationsstadium** | | |
| Einsatz zur Kristallisation, g/Operation | 396.0 | 390.03 |
| davon, g/Operation | | |
| MCHES | 297.00 | 313.35 |
| DCHES | 27.72 | 5.40 |
| ES | 71.28 | 71.28 |
| **Gewonnen:** | | |
| Mutterlauge, g/Operation | 196.00 | 149.24 |
| davon, g/Operation | | |
| MCHES | 99.00 | 74.56 |
| DCHES | 26.72 | 4.40 |
| ES | 70.28 | 70.28 |
| Handelsübliche MCHES, g/Operation | 200.00 | 240.79 |
| davon, g/Operation | | |
| MCHES | 198.00 | 238.39 |
| DCHES | 1.00 | 1.20 |
| ES | 1.00 | 1.20 |
| **Verbrauchskennziffer** | | |
| Tonnen je 1 Tonne MCHES | | |
| Essigsäure | 1.18 | 0.98 |
| Chlor | 1.66 | 1.38 |
| Kristallisationsleistung, g/Operation | 200.00 | 240.79 |

Tabelle 2

| Vers. Nr. | Durchm. cm | Fikt.Zufuhr- Geschw.für MCHES, m/s | Gehalt, gew. % | | Verbrauch Katalysator g |
|---|---|---|---|---|---|
| | | | DCHES | ES | |
| Beispiel 1 Prototyp | - - | - | 1,18 | 2,80 | 1,5 |
| Beispiel 2 | | | | | |
| 1. | 0,5 | 4,95 | 4,27 | 0,22 | 0,0 |
| 2. | 0,75 | 2,22 | 2,41 | 0,36 | 0,0 |
| 3. | 1,0 | 1,24 | 1,16 | 0,47 | 0,0 |
| 4. | 2,0 | 0,31 | 0,82 | 0,52 | 0,0 |
| 5. | 3,0 | 0,14 | 0,61 | 0,56 | 0,0 |
| 6. | 4,0 | 0,078 | 0,75 | 1,41 | 0,0 |
| 7. | 5,0 | 0,049 | 0,62 | 2,00 | 0,0 |
| 8. | 6,0 | 0,035 | 0,59 | 3,40 | 0,0 |
| 9. | 7,0 | 0,029 | 0,61 | 4,28 | 0,0 |

**Patentansprüche**

1.  Verfahren zur Herstellung von Monochloressigsäure (MCHES) durch Chlorierung von Essigsäure, wobei ein MCHES-Rohling erzeugt wird und aus dem Rohling durch Kristallisation und Zentrifugierung MCHES agbetrennt wird, dadurch gekennzeichnet, daß

vor der Kristallisation eine katalytische Hydrogenolyse durchgeführt wird, wobei diese in einem mit Gasaufzug (Airlift) ausgestatteten Apparat oder an einem Festbettkatalysator im Dünnschichtbetrieb vorgenommen wird.

**2.** Verfahren nach Anspruch 1, dadurch gekennzeichnet, daß
in der Hydrogenolyse ein Molverhältnis DCHES : $H_2$ = 1 : 7 eingestellt wird.

**3.** Verfahren nach Anspruch 2, dadurch gekennzeichnet, daß
als Katalysator Palladium auf Aktivkohle eingesetzt wird, insbesondere in einer Konzentration zwischen 0,5 und 2 gew. %.

**4.** Verfahren nach Anspruch 3, dadurch gekennzeichnet, daß
die Hydrogenolyse bei einer Temperatur zwischen 125 und 140 Grad Celsius durchgeführt wird.

**5.** Verfahren nach einem der Ansprüche 1 bis 4,
dadurch gekennzeichnet, daß
der Hydrogenolyse-Prozeß in einem Festbett-Katalysator im Dünnschichtbetrieb bei einer Zufuhrgeschwindigkeit des MCHES-Rohlings zwischen 0,049 und 1,24 m/s, insbesondere zwischen 0,1 und 0,5 m/s erfolgt.

**Claims**

**1.** Method of producing monochloroacetic acid (MCHES) by chlorinating acetic acid, an MCHES blank being produced, and MCHES being separated from the blank by crystallisation and centrifuging, characterised in that a catalytic hydrogenolysis is accomplished prior to the crystallisation, said hydrogenolysis being carried out in an apparatus provided with gas lift (airlift) or on a fixed-bed catalyst in the thin-layer operation.

**2.** Method according to claim 1, characterised in that a molar ratio of DCHES : $H_2$ is set at 1 : 7 in the hydrogenolysis.

**3.** Method according to claim 2, characterised in that palladium on active carbon is used as the catalyst, more especially in a concentration between 0.5 and 2 % by wt.

**4.** Method according to claim 3, characterised in that the hydrogenolysis is accomplished at a temperature between 125 and 140 degrees Celsius.

**5.** Method according to one of claims 1 to 4, characterised in that the hydrogenolysis process is effected in a fixed-bed catalyst in the thin-layer operation at a feed rate for the MCHES blank of between 0.049 and 1.24 m/s, more especially between 0.1 and 0.5 m/s.

**Revendications**

**1.** Procédé de préparation d'acide monochloroacétique (AMCHA) par chloration d'acide acétique, dans lequel on produit un produit intermédiaire de AMCHA et on sépare le AMCHA du produit intermédiaire par cristallisation et centrifugation, caractérisé en ce qu'on effectue une hydrogénolyse catalytique avant la cristallisation, celle-ci étant effectuée dans un appareil équipé d'un extracteur à air comprimé (Airlift) ou sur un catalyseur à lit fixe fonctionnant en couche mince.

**2.** Procédé selon la revendication 1, caractérisé en ce qu'on établit un rapport molaire de ADCHA : $H_2$ = 1 : 7 pendant l'hydrogénolyse.

**3.** Procédé selon la revendication 2, caractérisé en ce qu'on utilise, comme catalyseur, du palladium sur du charbon actif, en particulier à une concentration entre 0,5 et 2 % en poids.

**4.** Procédé selon la revendication 3, caractérisé en ce qu'on effectue l'hydrogénolyse à une température entre 125 et 140 degrés Celsius.

5. Procédé selon l'une des revendications 1 à 4, caractérisé en ce que le procédé d'hydrogénolyse se déroule dans un catalyseur à lit fixe fonctionnant en couche mince à une vitesse d'amenée du produit intermédiaire de AMCHA entre 0,049 et 1,24 m/s, en particulier entre 0,1 et 0,5 m/s.

ES(Essigsäure) →

Chlor →

**Essigsäure-Chlorierungs-Stadium**

MCHES-Rohling →

**Kristallisations-Stadium**

Rezyclus Mutterlauge

**Zentrifugier-Stadium**

handelsübliche MCHES

Abb. 1

ES(Essigsäure) →

Chlor →

┌─────────────────────┐
│ Essigsäure-Chlorier- │ — MCHES-Rohling →
│ Stadium              │
└─────────────────────┘

┌──────────────────┐
│ Hydrohgenolyse-  │
│ Stadium          │
└──────────────────┘

Rezyclus
Mutterlauge

┌──────────────────┐
│ Zentrifugier-    │
│ Stadium          │
└──────────────────┘

┌──────────────────┐
│ Kristallisations-│
│ Stadium          │
└──────────────────┘

handelsübliche
MCHES →

Abb. 2

Abb.3

МХУК
MCHES

Водород
Wasserstoff

Катализатор
Katalysator

Abgase
Абгазы

МХУК
MCHES

Abb. 4